# EUROPEAN PATENT APPLICATION

(11) **EP 4 516 111 A1**
(43) Date of publication of application: **05.03.2025**
(21) Application number: 23796513.2
(22) Date of filing: 27.04.2023
(51) Int. Cl.: A23J 3/34, A23J 3/00, A23J 3/16, A23J 3/18, A23L 13/00

(54) **PRODUCTION METHOD FOR PROCESSED PLANT PROTEIN FOOD AND PROCESSED PLANT PROTEIN FOOD**

(30) Priority: 28.04.2022 JP 2022075377
(71) Applicant: AJINOMOTO CO., INC., Chuo-ku Tokyo 104-8315 (JP)
(72) Inventor: SAZA, Takao, Fukuoka-shi, Fukuoka 812-0013 (JP); KAWATOU, Hwagyun, Kawasaki-shi, Kanagawa 210-8681 (JP)
(74) Representative: Strehl Schübel-Hopf & Partner
(86) International application number: PCT/JP2023/016752
(87) International publication number: WO 2023/210778

(57) **Abstract**

The present invention relates to a method for producing a plant-derived protein processed food, including swelling a porous granular plant-derived protein with water or a seasoning liquid, and then adding a powdered plant-derived protein and transglutaminase and kneading the mixture, and a plant-derived protein processed food containing a composition containing a porous granular plant-derived protein and a powdered plant-derived protein, the composition being acted on by transglutaminase.

The present invention makes it possible to provide a plant-derived protein processed food having a texture equivalent to that of meat, particularly steak meat or sliced meat.

## Description

### [Technical Field]

The present invention relates to production methods of plant-derived protein processed foods having a texture equivalent to that of meat, and plant-derived protein processed foods having a texture equivalent to that of meat.

### [Background Art]

As the global population increases, meat consumption is expected to increase, the increase in greenhouse gas emissions from animal husbandry has become a problem, and, given the health-consciousness in recent years, meat substitute foods that use plant-derived materials and have a texture similar to that of meat have attracted attention as meat substitute foods. As such meat substitute foods, granular soybean protein and the like that have been processed into a fibrous form by using a twin-screw extruder with a cooling die are on the market.

However, although the above-mentioned granular soybean protein has a fiber feeling similar to that of meat, it cannot be said to have a texture equivalent to that of meat in terms of elastic texture, juicy feel and the like.

Various attempts have also been made to improve the texture of the above-mentioned plant-derived protein processed foods such as soybean protein and the like. For example, a technique has been proposed in which transglutaminase acts on an emulsion of plant-derived protein and fats and oils, or an emulsion of plant-derived protein and fats and oils treated with transglutaminase is hydrated with an aqueous transglutaminase solution to produce a tissue-like plant-derived protein (Patent Literature 1), and a technique has been proposed in which transglutaminase, glucose oxidase, and phospholipase are added to a starting material containing plant-derived proteins to produce meat processed foods such as a hamburger steak (Patent Literature 2).

However, the technique described in Patent Literature 1 is mainly applied to ganmodoki, tofu steak, soybean protein ham, soybean protein sausage and the like, and the technique described in Patent Literature 2 is mainly applied to hamburger steak, and a plant-derived protein processed food having a texture equivalent to that of steak meat or sliced meat has not yet been obtained.

Therefore, the development of plant-derived protein processed foods that reproduce the texture of steak meat or sliced meat has been desired.

### [Citation List]

### [Patent Literatures]

[Patent Literature 1]
   JP 10-056976 A
[Patent Literature 2]
   WO 2017/154992

### [Summary of Invention]

### [Technical Problem]

The present invention thus aims to provide a method for producing a plant-derived protein processed food having a texture equivalent to that of meat, particularly steak meat and sliced meat, and to provide a plant-derived protein processed food having a texture equivalent to that of meat, such as steak meat and sliced meat.

### [Solution to Problem]

The present inventors have conducted intensive studies in an attempt to solve the above-mentioned problems and found that the elastic texture and juicy feel of meat, particularly the texture of steak meat and sliced meat, can be well reproduced in addition to the fiber feeling of meat, by swelling porous granular plant-derived protein with water or the like, then adding powdered plant-derived protein and transglutaminase, and kneading them. They have conducted further studies and completed the present invention.

That is, the present invention relates to the following.
[1] A method for producing a plant-derived protein processed food, comprising swelling a porous granular plant-derived protein with water or a seasoning liquid, and then adding a powdered plant-derived protein and transglutaminase and kneading the mixture.
[2] The production method of [1], wherein the porous granular plant-derived protein is a porous granular material comprising soybean protein, or a porous granular material prepared using soybean protein and a plant body comprising a protein as raw materials.
[3] The production method of [1] or [2], wherein the average particle size of the porous granular plant-derived protein is 1 mm to 100 mm.
[4] The production method of any of [1] to [3], wherein the bulk density of the porous granular plant-derived protein is 30 g/L to 300 g/L.
[5] The production method of any of [1] to [4], wherein the powdered plant-derived protein is a powder containing soybean protein extracted, concentrated or separated from defatted soybeans.
[6] The production method of any of [1] to [5], wherein the powdered plant-derived protein is added in an amount of 10 to 45 parts by weight per 100 parts by weight of the dry weight of the porous granular plant-derived protein.
[7] The production method of any of [1] to [6], wherein the transglutaminase is a calcium-independent transglutaminase derived from a microorganism.
[8] The production method of any of [1] to [7], wherein the transglutaminase is added in an amount such that the enzymatic activity of transglutaminase per 100 g of the dry weight of the porous granular plant-derived protein is 0.1 U to 1000 U.
[9] The production method of any of [5] to [8], wherein powdered gluten is further added as the powdered plant-derived protein.
[10] The production method of [9], wherein the powdered gluten is added in an amount of 1 to 40 parts by weight per 100 parts by weight of the dry weight of the porous granular plant-derived protein.
[11] The production method of any of [1] to [10], wherein glucose oxidase is further added.
[12] The production method of [11], wherein the glucose oxidase is added in an amount of added is an amount such that the enzyme activity of glucose oxidase per 100 g of the dry weight of the porous granular plant-derived protein is 0.1 U to 1000 U.
[13] A plant-derived protein processed food comprising a composition comprising a porous granular plant-derived protein and a powdered plant-derived protein, the composition being acted on by transglutaminase.
[14] The food of [13], wherein the porous granular plant-derived protein is a porous granular material comprising soybean protein or a porous granular material comprising soybean protein and a plant body containing a protein.
[15] The food of [13] or [14], wherein the average particle size of the porous granular plant-derived protein is 1 mm to 100 mm.
[16] The food of any of [13] to [15], wherein the bulk density of the porous granular plant-derived protein is 30 g/L to 300 g/L.
[17] The food of any of [13] to [16], wherein the content of the porous granular plant-derived protein is 1% by weight to 75% by weight with respect to the total amount of a plant-derived processed food.
[18] The food of any of [13] to [17], wherein the powdered plant-derived protein is a powder containing soybean protein extracted, concentrated or separated from defatted soybeans.
[19] The food of any of [13] to [18], wherein the content of the powdered plant-derived protein is 0.1% by weight to 22% by weight with respect to the total amount of the plant-derived processed food.
[20] The food of [18] or [19], wherein powdered gluten is further contained as the powdered plant-derived protein.
[21] The food of [20], wherein the content of the powdered gluten is 0.01% by weight to 20% by weight with respect to the total amount of the plant-derived processed food.
[22] The food of any of [13] to [21], wherein the composition comprising a porous granular plant-derived protein and a powdered plant-derived protein is a composition further acted on by glucose oxidase in addition to transglutaminase.

### [Advantageous Effects of Invention]

The present invention can provide a method for producing a plant-derived protein processed food having a texture equivalent to that of meat.

The present invention can also provide a plant-derived protein processed food having a texture equivalent to that of meat.

The present invention can well reproduce the elastic texture and juicy feel of meat, particularly the texture of steak meat and sliced meat, in addition to the fiber feeling of meat, in plant-derived protein processed foods.

### [Description of Embodiments]

The present invention provides a method for producing a plant-derived protein processed food (hereinafter to be also referred to as "the production method of the present invention" in the present specification).

The production method of the present invention includes swelling a porous granular plant-derived protein with water or a seasoning liquid, then adding a powdered plant-derived protein and transglutaminase, and kneading them.

The "porous granular plant-derived protein" used in the production method of the present invention refers to a porous granular material containing plant-derived protein, and is prepared by granulating a plant-derived protein extracted or separated or concentrated from a plant body containing a protein, by extrusion molding or the like, and drying same.

As the "plant body containing a protein" used in the preparation of the porous granular plant-derived protein, any edible organ or part of a plant that contains protein can be used without any particular limitation. Examples include seeds of beans such as soybean, green pea, broad bean, mung bean, and chickpea, seeds of wheat, rye and the like, asparagus stalk, broccoli inflorescence and stalk, green soybean (immature soybean seed), Brussels sprout, corn seed, and fruits such as buckwheat, avocado, and banana, and the like.

From the aspects of nutritional value and providing a texture similar to that of meat, seeds of beans are preferably used as the plant body containing a protein, and soybean is more preferably used.

In the production method of the present invention, as the porous granular plant-derived protein, a porous granular material containing soybean protein, or a porous granular material prepared using soybean protein and a plant body containing a protein as raw materials can be preferably used.

The porous granular material containing soybean protein is prepared by granulating, by extrusion molding, followed by drying, defatted soybeans, which are obtained by removing the seed coat from soybeans (soybean (Glycine max) seeds) and removing fats and oils, or a concentrated soybean protein obtained by washing the aforementioned defatted soybeans with alcohol or acid to remove sugars and ash.

Also, the porous granular material prepared using soybean protein and a plant body containing protein as the raw materials is prepared as a granular material by mixing the soybean protein with the above-mentioned plant body containing protein, granulating the mixture by extrusion molding, and then drying same. As the soybean protein, a protein concentrated, extracted, or separated from defatted soybeans, that is, a concentrated protein obtained by washing defatted soybeans with alcohol or acid to remove sugars and ash, a protein extracted from defatted soybeans with water or alkali, or a protein separated from defatted soybeans by extraction with water or alkali and precipitation with acid can be used. As the plant body containing protein, the seeds of beans such as soybeans, green peas, and broad beans can be preferably used.

For the purpose of the present invention, a porous granular material prepared using soybean protein and a plant body containing a protein as the raw materials is preferably used as the porous granular plant-derived protein, and a granular material prepared by mixing soybean protein separated from defatted soybeans with soybean whole-wheat flour, sprouted green peas, and sprouted soybeans as the plant body containing the protein, extrusion molding the mixture using an extruder, and drying the mixture is more preferably used.

The protein content of the porous granular plant-derived protein used in the production method of the present invention is preferably 50% by weight or more, more preferably 50% to 90% by weight.

The average particle size of the porous granular plant-derived protein is preferably 1 mm to 100 mm, more preferably 1 mm to 15 mm. The average particle size of the porous granular plant-derived protein is measured by a sieving method.

Furthermore, the porous granular plant-derived protein preferably has a bulk density of 30 g/L to 300 g/L, more preferably 75 g/L to 250 g/L, further preferably 75 g/L to 200 g/L, further more preferably 90 g/L to 200 g/L, as measured by the measurement method described below.

The protein content, average particle size, and bulk density of the porous granular plant-derived protein may vary depending on the preparation method of the soybean protein used as the raw material, the type of plant body containing the protein, and the conditions of extrusion molding, drying, and the like. An example of a porous granular plant-derived protein exhibiting the above-mentioned protein content, average particle size, and bulk density is a granular material obtained by using a powdered separated soybean protein described below as the soybean protein, adding and mixing soybean whole-wheat flour, sprouted soybeans, and sprouted green peas as plant bodies containing the protein to the aforementioned soybean protein, extrusion molding the mixture using a twin-screw extruder, and drying the mixture.

In the production method of the present invention, the porous granular plant-derived protein may be one selected from porous granular plant-derived proteins having different protein contents, average particle sizes, bulk densities, and the like, and used alone, or two or more selected therefrom may be used in combination.

In addition, the porous granular plant-derived protein may be produced using general separation and purification methods for soybean proteins, and general kneading and granulation techniques for powders and granules. Products commercially available as porous granular soybean proteins from various companies may also be used.

The "powdered plant-derived protein" used in the production method of the present invention is a powder obtained by drying plant-derived protein that has been extracted, concentrated, or separated from a plant body containing a protein.

As the plant body containing a protein, the same plants as those used to prepare porous granular plant-derived protein may be used. Seeds of beans such as soybeans, green peas, and broad beans, seeds of plants belonging to the Triticum genus such as wheat, and seeds of plants belonging to the Secale genus such as rye are preferably used.

From the viewpoint of the binding property of plant-derived proteins, powdered proteins obtained from soybeans and powdered gluten obtained from wheat or rye are more preferably used as powdered plant-derived proteins.

As the powdered proteins obtained from soybeans, an extracted soybean protein-containing powder (protein content: about 60% by weight) obtained by removing seed coats and fats and oils from soybeans, extracting the defatted soybeans with water or alkali, neutralizing the extract, drying same by spray drying or the like, and powdering same; a concentrated soybean protein-containing powder (protein content: about 65% by weight) obtained by washing the aforementioned defatted soybeans with alcohol or acid, removing sugars and ash to concentrate the protein, and drying and powdering the protein; and a separated soybean protein-containing powder (protein content: about 90% by weight) obtained by extracting the aforementioned defatted soybeans with water or alkali, performing precipitation with acid, neutralization with alkali to separate protein, and drying and powdering the protein by spray drying or the like are preferably used, and the aforementioned separated soybean protein-containing powder is more preferably used.

In the present invention, the powdered protein obtained from soybeans may be soybean protein extracted, concentrated, separated, and the like from defatted soybeans and the like, as described above, followed by drying and powdering, or commercially available products provided by various companies may also be used.

The powdered gluten is obtained by kneading wheat flour, rye flour, etc. with water to generate gluten through a reaction between gliadin and glutenin in the presence of water, extracting the gluten, and drying same by spray drying or the like to obtain a powder. Gliadin and glutenin are storage proteins present in the endosperm of seeds such as wheat and rye.

In the present invention, the powdered gluten may be obtained by kneading wheat flour, etc. with water to generate gluten, and extracting, drying, and powdering the gluten, or commercially available products provided by various companies may also be used.

In the production method of the present invention, one type of powdered plant-derived protein may be selected from various types of powdered plant-derived proteins that are different in the types of plant body containing the protein, the production method, the protein content, and the like, and used alone, or two or more selected therefrom may be used in combination.

In addition, the powdered plant-derived protein can be produced and used according to general separation and purification methods and general powdering techniques for plant-derived proteins. As described above, products provided by various companies, such as commercially available products of powdered soybean protein, powdered gluten, and the like can also be used.

In the production method of the present invention, it is preferable to use a combination of the above-mentioned soybean protein-containing powder and powdered gluten as the powdered plant-derived protein, because this can impart elastic texture and a texture similar to that of meat to the produced plant-derived protein processed foods.

The transglutaminase used in the production method of the present invention is a transferase (protein-glutamine γ-glutamyltransferase) that catalyzes the reaction of condensing the amino group of a glutamine residue in a protein with a primary amine, transferring the substituent on the amine to the glutamine residue, and generating ammonia. Generally, the amino group of a lysine residue in a protein is used as the primary amine, and the transglutaminase acts as a cross-linking enzyme.

As the transglutaminase, calcium-independent transglutaminase derived from a microorganism is preferably used.

As calcium-independent transglutaminase derived from a microorganism, transglutaminase produced by an actinomycete belonging to the genus Streptomyces can be mentioned. It can be obtained according to the method described in Japanese Patent No. 2572716, but commercially available products provided by Ajinomoto Co., Inc., and others can also be used.

In the production method of the present invention, first, water or a seasoning liquid is added to the porous granular plant-derived protein to cause swelling thereof. The seasoning liquid can be prepared by adding a general seasoning described later to water for dissolution.

The amount of water or seasoning liquid added to the porous granular plant-derived protein is appropriately set as an amount that can sufficiently swell the protein depending on the degree of porosity of the porous granular plant-derived protein. It is preferably 1 to 5 times, more preferably 2 to 3 times, of the dry weight of the porous granular plant-derived protein.

The above-mentioned step of swelling the porous granular plant-derived protein is generally performed in water or seasoning liquid at about 5°C to 60°C, preferably about 10°C to 50°C, for about 30 min to overnight, preferably about 1 hr to overnight.

Then, a powdered plant-derived protein and transglutaminase are added to the swollen porous granular plant-derived protein, water is added and the mixture is kneaded.

It is preferable to add and spread the powdered plant-derived protein and transglutaminase evenly over the entire surface of the swollen porous granular plant-derived protein.

The amount of the powdered plant-derived protein to be added is preferably 10 parts by weight to 45 parts by weight, more preferably 12 parts by weight to 42 parts by weight, per 100 parts by weight of the dry weight of the porous granular plant-derived protein.

When the amount of the powdered plant-derived protein added exceeds 50 parts by weight per 100 parts by weight of the dry weight of the porous granular plant-derived protein, the binding property of the plant-derived protein may decrease.

In addition, in the production method of the present invention, when a powder containing soybean protein and powdered gluten are used in combination as the powdered plant-derived protein, the amount of powdered gluten to be added is preferably 1 to 40 parts by weight, more preferably 10 to 30 parts by weight, per 100 parts by weight of the dry weight of the porous granular plant-derived protein.

Transglutaminase is added such that the enzymatic activity of transglutaminase per 100 g in dry weight of the porous granular plant-derived protein is preferably 0.1 units (U) to 1000 U, more preferably 1 U to 500 U, further preferably 1 U to 200 U.

The enzymatic activity of transglutaminase can be measured and calculated, for example, by the hydroxamate method. That is, the enzyme activity can be calculated by reacting benzyloxycarbonyl-L-glutaminylglycine with hydroxylamine as substrates, forming an iron complex of the hydroxamic acid produced in the aforementioned reaction, in the presence of trichloroacetic acid, measuring the absorbance at 525 nm, and determining the amount of hydroxamic acid produced from a calibration curve. In the present specification, the amount of enzyme that produces 1 µmol of hydroxamic acid per minute at 37°C and pH=6.0 is defined as 1 U (see JP S64-027471 A).

In the production method of the present invention, it is preferable to further add glucose oxidase in addition to transglutaminase from the aspect of improving the texture of the obtained plant-derived protein processed foods.

Glucose oxidase is an enzyme that catalyzes the reaction of oxidizing β-D-glucose to D-glucono-1,5-lactone and producing hydrogen peroxide. The produced D-glucono-1,5-lactone is non-enzymatically hydrolyzed to gluconic acid.

Examples of glucose oxidase include, but are not limited to, those derived from microorganisms such as filamentous fungi (Aspergillus aculeatus, Aspergillus niger, Penicillium genus). In the present invention, commercially available enzyme preparations provided by Shin Nippon Chemical Industry Co., Ltd. and the like can be used.

Glucose oxidase is added such that the enzyme activity of glucose oxidase is preferably 0.1 U to 1000 U, more preferably 1 U to 500 U, further preferably 1 U to 200 U, per 100 g of dry weight of the porous granular plant-derived protein.

The enzyme activity of glucose oxidase can be calculated by using glucose as a substrate, allowing glucose oxidase to act in the presence of oxygen to generate hydrogen peroxide, which is then acted on by peroxidase in the presence of aminoantipyrine and phenol to generate a quinoneimine dye, measuring the absorbance at 500 nm, and determining the amount of the quinoneimine dye from a calibration curve. In the present specification, the amount of enzyme that oxidizes 1 µmol of glucose per minute at 37°C and pH=7.0 is defined as 1 U.

In the production method of the present invention, in addition to the porous granular plant-derived protein, powdered plant-derived protein, and transglutaminase, and further, glucose oxidase, it is also possible to add general food additives such as seasonings such as salt, sugar, soy sauce, amino acid salts, nucleic acids, yeast extract, and meat extract; starch and processed starch; animal proteins such as egg white, gelatin, and casein; hydrolysates and partial hydrolysates of the aforementioned animal proteins; pH adjusters such as organic acid salts (fumarate, etc.); chelating agents such as polymerized phosphates; colorants; acidulants; and flavorings, within a range that does not impair the characteristics of the present invention.

The amount of water to be added during kneading is generally 10 to 50 parts by weight per 100 parts by weight in dry weight of the porous granular plant-derived protein.

In addition, the kneading is performed using a kneading machine such as a kneader, a twin-screw kneader, and the like, generally at 0°C to 50°C, preferably 4°C to 30°C, generally for 1 min to 30 min, preferably 2 min to 20 min.

The production method of the present invention may further include steps that may be included in general production methods of processed meat foods, such as degassing, compressing, molding, slicing, heating, sterilization, and packaging of kneaded products. Such steps may be performed under conditions and by methods generally used in the production of processed meat foods.

The production method of the present invention preferably includes a step of degassing or compressing, or degassing and compressing, after the above-mentioned kneading. By performing degassing or compressing, or degassing and compressing, the binding property of the plant-derived protein can be improved, and the texture of the plant-derived protein processed food can be made closer to that of meat.

The degassing or compressing, or degassing and compressing of the kneaded product can be performed by placing the kneaded product in a bag for molding, and using a vacuum packaging machine, a degassing sealer, or the like.

The production method of the present invention can provide a plant-derived protein processed food that has good elastic texture and juicy feel in addition to a fiber feeling similar to that of meat. The production method of the present invention can particularly reproduce the texture of steak meat and sliced meat.

The production method of the present invention is therefore preferably used in the production of plant-derived meat substitute foods.

The present invention also provides a plant-derived protein processed food (hereinafter also referred to as the "food of the present invention" in the present specification).

The food of the present invention is a composition containing a porous granular plant-derived protein and a powdered plant-derived protein, and contains a composition acted on by transglutaminase.

The "porous granular plant-derived protein" and "powdered plant-derived protein" contained in the composition contained in the food of the present invention are as described above for the production method of the present invention. The transglutaminase that acts on the composition containing the porous granular plant-derived protein and the powdered plant-derived protein is also as described above for the production method of the present invention.

The composition contained in the food of the present invention preferably contains powdered soybean protein and powdered gluten as the powdered plant-derived protein. By containing powdered soybean protein and powdered gluten as the powdered plant-derived protein, the food of the present invention is imparted well with an elastic texture and a texture closer to that of meat.

The "powdered soybean protein" and "powdered gluten" contained in the composition contained in the food of the present invention are as described above for the production method of the present invention.

Furthermore, from the aspect of the effect of improving the texture of the food of the present invention, it is preferable that the composition contained in the food of the present invention is one in which glucose oxidase has acted in addition to transglutaminase. The aforementioned glucose oxidase is as described above for the production method of the present invention.

The content of the porous granular plant-derived protein in the food of the present invention is preferably 1% by weight to 75% by weight, more preferably 5% by weight to 50% by weight, further preferably 10% by weight to 30% by weight, with respect to the total amount of the food of the present invention.

The content of the powdered plant-derived protein in the food of the present invention is preferably 0.1% by weight to 22% by weight, more preferably 0.5% by weight to 20% by weight, further preferably 1% by weight to 10% by weight, with respect to the total amount of the food of the present invention.

The content of the powdered gluten in the food of the present invention is preferably 0.01% by weight to 20% by weight, more preferably 0.1% by weight to 15% by weight, further preferably 1% by weight to 10% by weight, with respect to the total amount of the food of the present invention.

The composition contained in the food of the present invention is formed by the action of transglutaminase in an amount such that the enzymatic activity per 100 g of the total content of the porous granular plant-derived protein and powdered plant-derived protein is preferably 0.1 U to 1000 U, more preferably 1 U to 500 U, further preferably 1 U to 200 U.

When the composition contained in the food of the present invention is one in which glucose oxidase has acted in addition to transglutaminase, the composition contained in the food of the present invention is one in which glucose oxidase in an amount such that the enzyme activity per 100 g of the total content of the porous granular plant-derived protein and powdered plant-derived protein is preferably 0.1 U to 1000 U, more preferably 1 U to 500 U, further preferably 1 U to 200 U; has acted.

The composition contained in the food of the present invention can contain general food additives in addition to the porous granular plant-derived protein and powdered plant-derived protein, before or after the transglutaminase or further glucose oxidase act, as long as the characteristics of the present invention are not impaired.

Furthermore, the food of the present invention is a composition containing a porous granular plant-derived protein and a powdered plant-derived protein, and can contain general food additives in addition to the composition in which transglutaminase or further glucose oxidase has acted, as long as the characteristics of the present invention are not impaired.

The general food additives that may be contained in the food of the present invention are as described above for the production method of the present invention.

The food of the present invention can be produced by adding a powdered plant-derived protein and transglutaminase to a porous granular plant-derived protein allowed to swell by adding water or a seasoning liquid, mixing them, or further adding glucose oxidase, mixing them, adding water and kneading the mixture, and preferably compressing and degassing the mixture, followed by molding the mixture.

The methods and conditions for the above-mentioned swelling, mixing and kneading are as described above for the production method of the present invention.

The content of water in the food of the present invention is preferably 20% to 85% by weight, more preferably 40% to 80% by weight, further preferably 50% to 75% by weight, with respect to the total amount of the food.

The food of the present invention can be formed into a lump such as an oval sphere, a block, or the like, and can be sliced or diced depending on the mode of use thereof, and can further be cooked by heating, and the like.

The food of the present invention has good elastic texture and juicy feel in addition to a fiber feeling similar to that of meat. In particular, the texture of steak meat, sliced meat; etc. can be reproduced well, even though it is a plant-derived protein processed food.

Therefore, the food of the present invention is suitable as a plant-derived meat substitute food.

### [Example]

Furthermore, the present invention is described in detail with reference to examples.

### [Examples 1-4, Comparative Example 1] Plant-derived protein processed food

(1) in Table 1 was immersed in (2) at room temperature for 60 min to allow same to swell, and then (3)-(6) were mixed and added, and allowed to adhere evenly to the surface of the swollen (1). Then, (7) was added, and the mixture was kneaded at room temperature for 15 min in a kneader mixer, and the mixture was filled in a propylene bag, degassed, compressed, and left standing overnight in a refrigerator to obtain the plant-derived protein processed foods of Examples 1-4 and Comparative Example 1.

The ingredients shown below were used for each component in Table 1.
(i) Porous granular plant-derived protein containing soybean protein: a mixture of soybean whole wheat flour made into a powder, a powder containing separated soybean protein separated and powdered from defatted soybeans, sprouted green peas, and sprouted soybeans in a paste form, which was extrusion formed in a twin-screw extruder to give granules, followed by drying (protein content: 53.2% by weight, particle size: 1 mm to 15 mm) was used.
(ii) Powdered plant-derived protein: the following powder containing separated soybean protein and powdered gluten were used.
(ii-1) Powder containing separated soybean protein: protein extracted from defatted soybeans with alkali, precipitated by adding acid, and separated, neutralized with alkali and spraydried (protein content: 90% by weight) was used.
(ii-2) Powdered gluten: commercially available wheat gluten powder was used.
(iii) Transglutaminase: "Activa TG" (Ajinomoto Co., Inc., 1,150 U/g) was used.
(iv) A mixed preparation of transglutaminase and glucose oxidase: "KS-STG-MS" (Ajinomoto Co., Inc., containing 23 U/g transglutaminase and 26 U/g glucose oxidase) was used.

**[Table 1]**

| component | | content (g) | | | | |
|---|---|---|---|---|---|---|
| | | Comp. Ex. 1 | Ex. 1 | Ex. 2 | Ex. 3 | Ex. 4 |
| (1) | porous granular plant-derived protein containing soybean protein | 21.54 | 21. 54 | 21.54 | 21. 54 | 21. 54 |
| (2) | immersion water | 63.59 | 63.59 | 63.59 | 63.59 | 63.59 |
| (3) | powder containing separated soybean protein | 5.13 | 5.13 | 5.13 | 5.13 | 5.13 |
| (4) | powdered gluten | | | 3.59 | | 3.59 |
| (5) | transglutaminase | | 0.02 | 0.02 | | |
| (6) | transglutaminase and glucose oxidase mixed preparation | | | | 1.03 | 1.03 |
| (7) | additional water | 5.12 | 5.12 | 5.12 | 5.12 | 5.12 |
| total | | 95.38 | 95.40 | 98.99 | 96.41 | 100.00 |

| | | | | | | |
|---|---|---|---|---|---|---|
| *; blank space in the Table indicates that the component is not contained | | | | | | |

Each of the plant-derived protein processed foods of Examples 1 to 4 and Comparative Example 1 was sliced, and the binding property of the plant-derived protein and the ease of slicing were evaluated by five panelists based on the following evaluation criteria. In addition, each sliced plant-derived processed food was fried in a frying pan, and the five panelists ate it and performed a sensory evaluation of the elastic texture and juicy feel based on the following evaluation criteria. The evaluation results were determined by discussion among the five panelists and are shown in Table 2.

### <Evaluation criteria>

(1) Binding property of plant-derived protein
   +++: very good
   ++: good
   +: slightly insufficient
   -: insufficient
(2) Ease of slicing
   +++: easy to slice
   ++: slicing is possible
   +: slicing is quite difficult
   -: slicing is not possible
(3) Elastic texture
   +++: elastic texture similar to that of meat
   ++: elastic texture quite similar to that of meat
   +: slightly weaker elastic texture than that of meat
   -: weak elastic texture compared to that of meat
(4) Juicy feel
   +++: juicy feel similar to that of meat
   ++: juicy feel quite similar to that of meat
   +: less juicy feel than that of meat
   -: no juicy feel compared to that of meat

**[Table 2]**

| evaluation item | Comparative Example 1 | Example 1 | Example 2 | Example 3 | Example 4 |
|---|---|---|---|---|---|
| binding property | - | +++ | +++ | +++ | +++ |
| ease of slicing | - | ++ | +++ | ++ | +++ |
| elastic texture | × | ++ | +++ | ++ | +++ |
| juicy feel | × | ++ | +++ | ++ | +++ |

| | | | | | |
|---|---|---|---|---|---|
| *; in the Table, "x" indicates that the evaluation could not be performed | | | | | |

As shown in Table 2, in the plant-derived protein processed food of Comparative Example 1, which was produced by simply adding a powder containing separated soybean protein as a powdered plant-derived protein to a porous granular plant-derived protein swollen with water and kneading the mixture, without adding either transglutaminase or glucose oxidase, the binding property of the plant-derived protein was insufficient, and when an attempt was made to slice the food, it crumbled and could not be sliced, making it impossible to evaluate the elastic texture and juicy feel.

In contrast, in the plant-derived protein processed food of Example 1, which was produced by adding a powder containing separated soybean protein as a powdered plant-derived protein to porous granular plant-derived protein swollen with water, adding transglutaminase, and kneading the mixture, and in the plant-derived protein processed food of Example 3, which was produced by adding a powder containing separated soybean protein as a powdered plant-derived protein to porous granular plant-derived protein swollen with water, adding transglutaminase and glucose oxidase, and kneading the mixture, the plant-derived proteins bonded very well and the foods were quite easy to slice. In addition, it was evaluated that the elastic texture and juicy feel thereof were quite similar to those of meat.

Furthermore, in the plant-derived protein processed food of Example 2, which was produced by adding a powder containing separated soybean protein as a powdered plant-derived protein and powdered gluten to a porous granular plant-derived protein swollen with water, further adding transglutaminase, and kneading the mixture, and the plant-derived protein processed food of Example 4, which was produced by adding a powder containing separated soybean protein as a powdered plant-derived protein and powdered gluten to a porous granular plant-derived protein swollen with water, further adding transglutaminase and glucose oxidase, and kneading the mixture, the binding property was very good, the foods could be easily sliced, and were evaluated to have an elastic texture and juicy feel similar to those of meat.

The above results show that in order to obtain good binding property in plant-derived protein processed foods, it is necessary to add powdered plant-derived protein and transglutaminase to porous granular plant-derived protein and knead the mixture.

It was also suggested that an elastic texture and juicy feel closer to those of meat can be obtained by adding a powder containing separated soybean protein and powdered gluten as a powdered plant-derived protein to porous granular plant-derived protein, adding transglutaminase and kneading, and further adding glucose oxidase and kneading.

### [Examples 5-7 and Comparative Examples 2 and 3] Plant-derived protein processed foods

Plant-derived protein processed foods were produced in the same manner as in the above-mentioned plant-derived protein processed food of Example 4 except that commercially available porous granular soybean proteins (commercial products A, B, and C) were used instead of the porous granular plant-derived protein containing soybean protein, and used as Examples 5 to 7. In addition, plant-derived protein processed foods were produced in the same manner using commercially available granular soybean proteins (commercial products D and E), and used as Comparative Examples 2 and 3.

The porous granular plant-derived protein used in the production of the plant-derived protein processed food of Example 4, the porous granular soybean proteins used in the production of the plant-derived protein processed foods of Examples 5 to 7, and the granular soybean proteins used in the production of the plant-derived protein processed foods of Comparative Examples 2 and 3 were each leveled with and filled in a 1 L stainless steel mug (volume=0.986 L), the weights were measured, and the bulk density of each was determined and is shown in Table 3 as mean±standard deviation (n=5).

The plant-derived protein processed foods of Examples 4 to 7 and the plant-derived protein processed foods of Comparative Examples 2 and 3 were also evaluated for the binding property in the same manner as the above-mentioned plant-derived protein processed foods of Examples 1 to 4 and Comparative Example 1, and the evaluation results are also shown in Table 3.

**[Table 3]**

| evaluation item | Ex. 4 | Ex. 5 | Ex. 6 | Ex. 7 | Comp. Ex. 2 | Comp. Ex. 3 |
|---|---|---|---|---|---|---|
| bulk density (g/L) of granular plant-derived protein | 109.7± 1.9 | 192.6± 4.6 | 161.2± 2.8 | 93.2± 3.2 | 343.4± 7.2 | 304.4± 3.8 |
| binding property of plant-derived protein | +++ | +++ | +++ | +++ | - | - |

As shown in Table 3, the bulk densities of the porous granular plant-derived protein used in the production of the plant-derived protein processed food of Example 4 and the porous granular soybean proteins used in the production of the plant-derived protein processed foods of Examples 5 to 7 were 109.7±1.9 g/L, 192.6±4.6 g/L, 161.2±2.8 g/L, and 93.2±3.2 g/L, respectively.

On the other hand, the bulk densities of the granular soybean proteins used in the production of the plant-derived protein processed foods of Comparative Examples 2 and 3 were 343.4±7.2 g/L and 304.4±3.8 g/L.

Also, as shown in Table 3, the plant-derived protein processed foods of Examples 5 to 7 showed good binding property of the plant-derived proteins, similar to the plant-derived protein processed food of Example 4; however, the plant-derived protein processed foods of Comparative Examples 2 and 3 did not show sufficient binding property of the plant-derived proteins.

The above results suggest that it is preferable to use a porous granular plant-derived protein that shows a bulk density of 300 g/L or less, more preferably 200 g/L or less, in the present invention.

In addition, from the aspect of obtaining good binding property, the bulk density of the porous granular plant-derived protein is particularly preferably 90 g/L to 200 g/L.

### [Industrial Applicability]

As described above in detail, the present invention can provide a method for producing a plant-derived protein processed food having a texture equivalent to that of meat.

The present invention can also provide a plant-derived protein processed food having a texture equivalent to that of meat.

The present invention can well reproduce the elastic texture and juicy feel of meat in addition to the fiber feeling of meat, particularly the texture of steak meat, sliced meat, and the like in a plant-derived protein processed food.

This application is based on a patent application No. 2022-075377 filed in Japan, the contents of which are incorporated in full herein.

## Claims

1. A method for producing a plant-derived protein processed food, comprising swelling a porous granular plant-derived protein with water or a seasoning liquid, and then adding a powdered plant-derived protein and transglutaminase and kneading the mixture.

2. The production method according to claim 1, wherein the porous granular plant-derived protein is a porous granular material comprising soybean protein, or a porous granular material prepared using soybean protein and a plant body comprising a protein as raw materials.

3. The production method according to claim 1 or 2, wherein the average particle size of the porous granular plant-derived protein is 1 mm to 100 mm.

4. The production method according to claim 1 or 2, wherein the bulk density of the porous granular plant-derived protein is 30 g/L to 300 g/L.

5. The production method according to claim 1 or 2, wherein the powdered plant-derived protein is a powder containing soybean protein extracted, concentrated or separated from defatted soybeans.

6. The production method according to claim 1 or 2, wherein the powdered plant-derived protein is added in an amount of 10 to 45 parts by weight per 100 parts by weight of the dry weight of the porous granular plant-derived protein.

7. The production method according to claim 1 or 2, wherein the transglutaminase is a calcium-independent transglutaminase derived from a microorganism.

8. The production method according to claim 1 or 2, wherein the transglutaminase is added in an amount such that the enzymatic activity of transglutaminase per 100 g of the dry weight of the porous granular plant-derived protein is 0.1 U to 1000 U.

9. The production method according to claim 5, wherein powdered gluten is further added as the powdered plant-derived protein.

10. The production method according to claim 9, wherein the powdered gluten is added in an amount of 1 to 40 parts by weight per 100 parts by weight of the dry weight of the porous granular plant-derived protein.

11. The production method according to claim 1 or 2, wherein glucose oxidase is further added.

12. The production method according to claim 11, wherein the glucose oxidase is added in an amount of added is an amount such that the enzyme activity of glucose oxidase per 100 g of the dry weight of the porous granular plant-derived protein is 0.1 U to 1000 U.

13. A plant-derived protein processed food comprising a composition comprising a porous granular plant-derived protein and a powdered plant-derived protein, the composition being acted on by transglutaminase.

14. The food according to claim 13, wherein the porous granular plant-derived protein is a porous granular material comprising soybean protein or a porous granular material comprising soybean protein and a plant body containing a protein.

15. The food according to claim 13 or 14, wherein the average particle size of the porous granular plant-derived protein is 1 mm to 100 mm.

16. The food according to claim 13 or 14, wherein the bulk density of the porous granular plant-derived protein is 30 g/L to 300 g/L.

17. The food according to claim 13 or 14, wherein the content of the porous granular plant-derived protein is 1% by weight to 75% by weight with respect to the total amount of a plant-derived processed food.

18. The food according to claim 13 or 14, wherein the powdered plant-derived protein is a powder containing soybean protein extracted, concentrated or separated from defatted soybeans.

19. The food according to claim 13 or 14, wherein the content of the powdered plant-derived protein is 0.1% by weight to 22% by weight with respect to the total amount of the plant-derived processed food.

20. The food according to claim 18, wherein powdered gluten is further contained as the powdered plant-derived protein.

21. The food according to claim 20, wherein the content of the powdered gluten is 0.01% by weight to 20% by weight with respect to the total amount of the plant-derived processed food.

22. The food according to claim 13 or 14, wherein the composition comprising a porous granular plant-derived protein and a powdered plant-derived protein is a composition further acted on by glucose oxidase in addition to transglutaminase.
